Europäisches Patentamt

European Patent Office

Office européen des brevets

(19)

(11) Numéro de publication: **0 295 968 B1**

(12) **FASCICULE DE BREVET EUROPEEN**

(45) Date de publication de fascicule du brevet: **04.05.94**

(51) Int. Cl.5: **C05F 11/08**, C12N 11/10, C12N 1/04

(21) Numéro de dépôt: **88400435.9**

(22) Date de dépôt: **25.02.88**

(54) **Engrais contenant des microorganismes et leurs procédés de fabrication.**

(30) Priorité: **27.02.87 FR 8702634**

(43) Date de publication de la demande:
**21.12.88 Bulletin 88/51**

(45) Mention de la délivrance du brevet:
**04.05.94 Bulletin 94/18**

(84) Etats contractants désignés:
**AT BE CH DE ES FR GB GR IT LI LU NL SE**

(56) Documents cités:
EP-A- 0 017 565       EP-A- 0 083 267
EP-A- 0 187 732       DE-A- 2 835 874
DE-A- 3 417 899       DE-C- 716 275
FR-A- 2 600 673       US-A- 2 313 057
US-A- 4 421 544       US-A- 4 605 622

(73) Titulaire: **LIPHA, LYONNAISE INDUSTRIELLE PHARMACEUTIOUE**
**34, rue Saint Romain**
**F-69379 Lyon Cedex 08(FR)**

(72) Inventeur: **Fages, Jacques**
**Ouartier Saguenes, Hauts de Cugnaux**
**F-31120 Portet Sur Garonne(FR)**
Inventeur: **Rigal, Jacques**
**172 Chemin Lapujade**
**F-31200 Toulouse(FR)**
Inventeur: **Mullard Daniel**
**5 Impasse Vie Vieille**
**F-31270 Cugnaux(FR)**

(74) Mandataire: **Polus, Camille et al**
**c/o Cabinet Lavoix**
**2, Place d'Estienne d'Orves**
**F-75441 Paris Cedex 09 (FR)**

EP 0 295 968 B1

Rank Xerox (UK) Business Services
(3.10/3.09/3.3.3)

# EP 0 295 968 B1

**Description**

La présente invention concerne des engrais contenant des microorganismes et leurs procédés de fabrication.

Il est connu que les plantes de grande culture entretiennent une microflore, constituée par exemple de bactéries fixatrices de l'azote associées à leurs racines, et que ces associations rhizosphériques ont un effet de promotion de la croissance végétale.

Par addition d'un inoculum d'une bactérie de la rhizosphère on observe une modification du développement racinaire (élongation, poids sec, degré de branchement, nombre de poils absorbants), une modification de la matière sèche totale, et une augmentation du rendement en grains. Ces effets sont probablement dus à une conjonction de plusieurs phénomènes tels que fixation d'azote, production d'hormones de croissance végétale, activité pectinolytique permettant une meilleure absorption minérale. L'influence de l'addition d'inocula de bactéries dans la rhizosphère de plantes est par exemple décrit dans l'article de Gaskins M.H., Albrecht S.L. et Hubbel D.H. Agric. Ecos. Environ.1984 (12), pp.99-116.

Les méthodes utilisées par les chercheurs: inoculation liquide ou sous forme de granulés indépendamment de toute autre pratique agricole sont difficilement réalisables aux champs du fait du surcroît de travail qu'elles imposeraient aux agriculteurs. On a proposé pour la culture des légumineuses de conditionner des inocula sur de la tourbe et d'enrober les semences à l'aide du produit obtenu, mais ce procédé présente l'inconvénient de mettre en contact direct les microorganismes avec les différents pesticides qui enrobent généralement les graines. Or ces pesticides présentent le plus souvent une forte toxicité vis-à-vis de la plupart des espèces bactériennes.

Il serait intéressant de pouvoir mélanger les engrais et les inocula de bactéries de la rhizosphère car on a montré que l'effet d'une inoculation était meilleur en présence de fertilisants minéraux et en particulier azotés. En effet, les plantes ayant une bonne nutrition minérale, présentent une forte exsudation racinaire qui favorise le développement de la microflore rhizosphérique. Cependant jusqu'à présent on se heurtait à ladifficulté que les inocula n'avaient pas, au contact de l'engrais, une viabilité suffisante.

Selon la présente invention on a trouvé une forme d'inoculum possédant une viabilité suffisante, au contact de l'engrais, pour une utilisation directe aux champs. Plus précisément la présente invention concerne un engrais contenant des microorganismes isolés de la rhizosphère conditionnés par microencapsulation dans une matrice de polysaccharide.

On connaît déja, notamment par les brevets EP-A-017.565 et EP-A-083.267, des procédés de microencapsulation de microorganismes dans une matrice de polysaccharide. Un autre procédé utilisable selon la présente invention consiste à:
- préparer une culture de microorganismes dans un fermenteur en présence d' oxygène,
- arrêter l'arrivée d'oxygène et ajouter, de préférence sous agitation, du polysaccharide au milieu de culture,
- appliquer sur la surface du mélange une surpression de 0,2 à 1,9 bar environ, selon la nature et la concentration du polysaccharide ajouté,
- introduire le mélange sous pression dans un dispositif comprenant au moins une buse calibrée ayant un diamètre de 0,1 à 0,8 mm environ,
- projeter le mélange, à l'aide de la buse, sous forme de gouttes dans une solution de sel minéral,
- rincer et sècher le gel obtenu de façon à obtenir un inoculum stockable.

Le sel minéral utilisé dans ce procédé peut etre notamment un sel, tel que chlorure ou sulfate, de fer, manganèse, aluminium, calcium, zinc ou cuivre.

Le microorganisme encapsulé se présente généralement sous forme d'une poudre beige clair pouvant être mélangée à tout engrais contenant de l'azote et/ou du phosphore et/ou du potassium. Elle est plus avantageusement mélangée aux engrais azotés tels que nitrate d'ammonium ou urée.

L'engrais est sous forme solide. On peut par exemple mélanger l'engrais granulé avec la poudre d'inoculum microencapsulé dans un tambour jusqu'à ce que le mélange soit homogène. Les quantités respectives d'engrais et d'inoculum microencapsulé utilisables sont généralement telles que le rapport en poids de l'engrais à l'inoculum microencapsulé soit compris entre 500 et 2.000 environ. On peut ensuite pulvériser sur les granulés d'engrais un produit d'enrobage tel qu'une amine, une cire naturelle ou une cire synthétique.

Le produit d'enrobage présente notamment l'avantage, dans la mise en oeuvre de la présente invention, de réaliser efficacement une adhésion homogène de l'inoculum. L'engrais selon l'invention peut ensuite être stocké.

Les exemples donnés ci-dessous à titre non limitatif permettront de mieux comprendre l'invention.

2

EXEMPLE 1

L'Azospirillum lipoferum, microorganisme isolé de la rhizosphère du maïs, est cultivé dans un milieu contenant, par litre :

| | |
|---|---|
| $MgSO_4$, $7H_2O$ | 200 mg |
| NaCl | 100 mg |
| $CaCl_2$ | 20 mg |
| $FeSO_4$, $7H_2O$ | 10 mg |
| $Na_2MoO_4$, $2H_2O$ | 2 mg |
| Extrait de levure | 3 mg |
| $KH_2PO_4$ | 400 mg |
| $K_2HPO_4$ | 600 mg |
| Glucose | 30 g |
| $NH_4Cl$ | 5 g |
| $H_2O$ | QSP litre |

Le débit d'air et la vitesse d'agitation sont tels que la concentration en oxygène dissout soit de 30%. Le pH de départ étant de 6,8, il évolue jusqu'à 7,1 puis est maintenu à cette valeur par addition de soude 1 N.

La microencapsulation de cet inoculum est effectuée avec une solution d'alginate de sodium à raison de 10 g/l. On agite le mélange à une vitesse de 600 t/min. On soumet la solution à une pression de 0,5 bar puis on la disperse dans une solution de $CaCl_2$ à 6 g/l.

On rince le gel obtenu à l'eau puis on le sèche par étuvage à une température de 30°C avec une humidité relative de 30%.

EXEMPLE 2

On mélange pendant 5 minutes environ une quantité A d'inoculum microencapsulé de l'exemple 1 avec une quantité B de nitrate d'ammonium granulé, dans un tambour de granulation. Le rapport pondéral B/A est de 1000. On pulvérise sur les granulés ainsi obtenus une quantité C d'une huile aminée commercialisée sous la dénomination LILAMINE AC 81L. Cette amine en figeant vient enrober les granulés de nitrate d'ammonium et on obtient une adhésion homogène de l'inoculum. Le rapport pondéral A/C est de 1.

On mesure ensuite le nombre des bactéries viables selon le protocole suivant :

-   2 g de nitrate d'ammonium sont dissous dans 500 ml d'eau distillée stérile. On recueille l'inoculum pulvérulent par filtration sur filtre de porosité 0,45 um.
-   le filtre est mis dans 50 ml de tampon phosphate ayant la composition suivante :

| | |
|---|---|
| $KN_2PO_4$ | 3,336 g |
| $Na_2HPO_4$ | 18,773 g |
| $H_2O$ | QSP 1 litre |

et ayant un pH de 7,2. Dans une solution tampon le polysaccharide est déréticulé.

On facilite cette opération en mettant le flacon utilisé 1 heure sur table agitante orbitale. On étale des dilutions décimales sur boîte de Petri et on procède à une numération des colonies apparues après incubation de 48 à 72 heures à la température de 35°C, température optimale de croissance de l'Azospirillum lipoferum.

On observe une viabilité de :

```
1,6 x 10⁹ cellules viables après 26 heures de stockage.
1,5 x 10⁹    "         "      "    3 jours         "
0,7 x 10⁹    "         "      "    9 jours         "
0,3 x 10⁹    "         "      "    1 mois          "
```

# EP 0 295 968 B1

**Revendications**

1. Composition d'engrais obtenue par mélange de microcapsules contenant des microorganismes isolés de la rhizosphère dans une matrice de polysaccharide avec un engrais solide.

2. Composition pour engrais selon la revendication 1, caractérisée en ce que ledit engrais solide est un engrais solide granulé.

3. Composition pour engrais selon la revendication 2, caractérisée en ce que le rapport pondéral dudit engrais solide granulé audit inoculum microencapsulé est compris entre 500 et 2000.

4. Composition pour engrais selon l'une quelconque des revendications précédentes, caractérisée en ce que ledit engrais solide est du nitrate d'ammonium.

5. Composition pour engrais selon l'une quelconque des revendications précédentes, obtenue par pulvérisation d'un produit d'enrobage sur le mélange de microcapsules et d'engrais solide.

6. Composition pour engrais selon la revendication 5, caractérisée en ce que le produit d'enrobage est choisi parmi une amine, une cire naturelle et une cire synthétique.

7. Composition pour engrais selon la revendication 6, caractérisée en ce que le produit d'enrobage est une huile aminée.

**Claims**

1. Fertilizer composition obtained by mixing microcapsules containing microorganisms isolated from the rhizosphere in a polysaccharide matrix with a solid fertilizer.

2. Fertilizer composition according to claim 1, characterized in that the said solid fertilizer is a granulated solid fertilizer.

3. Fertilizer composition according to claim 2, characterized in that the weight ratio of the said granulated solid fertilizer to the said microencapsulated inoculum is between 500 and 2,000.

4. Fertilizer composition according to any one of the preceding claims, characterized in that the said solid fertilizer is ammonium nitrate.

5. Fertilizer composition according to any one of the preceding claims, obtained by spraying a coating product over the mixture of microcapsules and solid fertilizer.

6. Fertilizer composition according to claim 5, characterized in that the coating product is chosen from an amine, a natural wax and a synthetic wax.

7. Fertilizer composition according to claim 6, characterized in that the coating product is an aminecontaining oil.

**Patentansprüche**

1. Düngemittel-Zubereitung erhalten durch Vermischen von Mikrokapseln, die aus der Rhizosphäre isolierte Mikroorganismen in einer Polysaccharidmatrix enthalten, mit einem festen Düngemittel.

2. Zubereitung für Düngemittel nach Anspruch 1, **dadurch gekennzeichnet,** daß das feste Düngemittel ein granuliertes festes Düngemittel ist.

3. Zubereitung für Düngemittel nach Anspruch 2, **dadurch gekennzeichnet,** daß das Gewichtsverhaltnis von festem, granuliertem Düngemittel zu dem mikroeingekapselten Inokulum zwischen 500 und 2000 liegt.

4

4. Zubereitung für Düngemittel nach einem der vorhergehenden Ansprüche, **dadurch gekennzeichnet,** daß das feste Düngemittel Ammoniumnitrat ist.

5. Zubereitung für Düngemittel nach einem der vorhergehenden Ansprüche, erhalten durch Aufsprühen eines Umhüllungsprodukts auf die Mischung aus den Mikrokapseln und dem festen Düngemittel.

6. Zubereitung für Düngemittel nach Anspruch 5, **dadurch gekennzeichnet,** daß das Umhüllungsprodukt aus Aminen, natürlichem Wachs und synthetischem Wachs ausgewählt ist.

7. Zubereitung für Düngemittel nach Anspruch 6, **dadurch gekennzeichnet,** daß das Umhüllungsprodukt ein aminhaltiges Öl ist.